# EUROPÄISCHE PATENTANMELDUNG

(11) **EP 0 548 738 A1**
(43) Veröffentlichungstag der Anmeldung: **30.06.1993**
(21) Anmeldenummer: 92121286.6
(22) Anmeldetag: 15.12.1992
(51) Int. Cl.: C07C 69/734, C07C 67/52

(54) **Verfahren zur Isolierung und Reinigung von Oxadimethacrylverbindungen**

(30) Priorität: 24.12.1991 DE 4142912
(71) Anmelder: BASF Aktiengesellschaft, D-67063 Ludwigshafen (DE)
(72) Erfinder: Besecke, Siegmund, Dr., W-3250 Hameln (DE); Deckers, Andreas, Dr., W-6700 Ludwigshafen (DE); Lauke, Harald, Dr., W-6800 Mannheim 1 (DE)

(57) **Zusammenfassung**

Isolierung und Reinigung von Oxadimethacrylverbindungen der allgemeinen Formel I

CH₂=C(A)CH₂-O-CH₂C(B)=CH₂ I

in der A und B ausgewählt sind aus der Gruppe aus -COOR¹, -COR¹, - CONR²R³ und -CN, und R¹, R² und R³ folgende Bedeutung haben:
- R¹: Wasserstoff, Alkyl, unsubstituiertes oder substituiertes Cycloalkyl und Cycloalkyl-alkyl, Hydroxy-alkyl, Amino-alkyl, N-Alkyl-amino-alkyl, N,N-Di-alkyl-amino-alkyl, unsubstituiertes oder substituiertes Aryl und Aryl-alkyl,
- R²,R³: Wasserstoff, Alkyl, unsubstituiertes oder substituiertes Cycloalkyl und Cycloalkyl-alkyl, unsubstituiertes oder substituiertes Aryl und Aryl-alkyl,
indem man sie aus ihren Lösungen, enthaltend mindestens eine flüssige Kohlenwasserstoffverbindung, ausfällt oder auskristallisiert.

## Beschreibung

Die Erfindung betrifft ein Verfahren zur Isolierung und Reinigung von Oxadimethacrylverbindungen der allgemeinen Formel I

CH₂=C(A)CH₂-O-CH₂C(B)=CH₂ I

in der A und B ausgewählt sind aus der Gruppe aus -COOR¹, -COR¹, -CONR²R³ und -CN, und R¹, R² und R³ folgende Bedeutung haben:
- R¹: Wasserstoff, Alkyl, unsubstituiertes oder substituiertes Cycloalkyl und Cycloalkyl-alkyl, Hydroxy-alkyl, Amino-alkyl, N-Alkyl-amino-alkyl, N,N-Di-alkyl-amino-alkyl, unsubstituiertes oder substituiertes Aryl und Aryl-alkyl,
- R²,R³: Wasserstoff, Alkyl, unsubstituiertes oder substituiertes Cycloalkyl und Cycloalkyl-alkyl, unsubstituiertes oder substituiertes Aryl und Aryl-alkyl.

Oxadimethacrylverbindungen I sind beispielsweise aus der US-A 4,889,948 und aus Polymer Preprints, American Chemical Society, Division of Polymer Chemistry 31(1) (1990) 503 bekannt.

Sie sind wegen ihrer Bifunktionalität als Monomerbausteine geschätzte Verbindungen, die vielfach Verwendung finden, beispielsweise als Monomere zur Herstellung von Homopolymeren, oder als Comonomere und Vernetzer. Man kann sie nach der US-A 4,889,948 ausgehend von Acrylverbindungen des Typs Acrylverbindung II, H₂C=C(A)H, als auch ausgehend von Alkoholen des Typs Alkohol III, H₂C=C(A)CH₂OH, erhalten. Die Reaktion der Alkohole des Typs Alkohol III zu den Oxadimethacrylverbindungen I wird dabei unter Erhitzen durchgeführt, wobei bei ein- bis zweitägigen Reaktionszeiten neben beträchtlicher Polymerisation der Monomere nur mäßige Ausbeuten an Oxadimethacrylverbindungen I erhalten werden.

Ausgehend von den Acrylverbindungen des Typs Acrylverbindung II, H₂C=C(A)H, die man in Gegenwart des tertiären Amins 1,4-Diazabicyclo-[2.2.2]-octan (DABCO®) mit Formaldehyd umsetzt, erhält man als Hauptprodukt nach der US-A 4,889,948 Alkohole des Typs Alkohol III, H₂C=C(A)CH₂OH. Die Oxadimethacrylverbindungen I entstehen dabei nur in geringen Mengen. Daneben entstehen höhere (Ether-)Homologe der Oxadimethacrylverbindungen I mit der allgemeinen Formel Ia

CH₂=C(A)CH₂-O-CH₂-[-O-CH₂]ₙ-C(A)=CH₂ Ia

in der mit n = 1 bis 4 über 90 % dieser Homologen erfaßt werden, und polymere Nebenprodukte. Neben dieser unspezifischen Reaktion sind weiter nachteilig die langen Reaktionszeiten (10 bis 20 Tage) sowie eine beträchtliche Polymerbildung bei Temperaturen oberhalb der Raumtemperatur.

Bei der Aufarbeitung des Rohproduktes ist man selbstverständlich bestrebt, möglichst wenig Produkt zu verlieren. Und bei der Weiterverarbeitung der Oxadimethacrylverbindungen I, beispielsweise zur Herstellung von Polymerisaten, versteht es sich von selbst, daß möglichst reine Verbindungen zur Verfügung stehen sollten.

In J.Dent.Res. 3 (1990) 69 wird die Isolierung des Dimethylesters der 2,2' [Oxybis(methylen)]bis-2-propensäure ("Oxadimethacrylsäure") durch eine aufwendige Kombination aus Extraktion, Destillation und anschließende säulenchromatographische Trennung beschrieben, wobei Ausbeuten von nur 15 bis 25 % erreicht werden.

In Polymer Preprints, American Chemical Society, Division of Polymer Chemistry 31(1) (1990) 503 wird die Reinigung des Rohgemisches durch Säulenchromatographie mit nachfolgender Umkristallisation aus Methanol beschrieben. Je nach eingesetzter Ausgangsverbindung werden Ausbeuten von 37 bis 82 % erreicht.

In der US-A 4,889,948 wird die direkte fraktionierte Destillation des Rohgemisches im Hochvakuum beschrieben. Um die gewünschte Reinheit zu erzielen, wird zusätzlich eine Umkristallisation in Methanol durchgeführt. Die dabei erhaltenen Produkte fallen in Ausbeuten von nur < 20 % an.

Eine weitere in der US-A 4,889,948 beschriebene Möglichkeit besteht darin, daß man das Reaktionsgemisch zunächst zur Entfernung der wasserlöslichen Bestandteile mit Wasser extrahiert. Die davon dann abgetrennte organische Phase wird anschließend mit Schwefelsäure behandelt. Danach destilliert man die leichtsiedenden Bestandteile ab, wäscht nochmals mit Wasser, und kristallisiert das Produkt schließlich aus Methanol. Die Ausbeuten bei diesem Verfahren liegen im Bereich von 10 bis 20 %.

Aufgabe der vorliegenden Erfindung war es daher, unter überwindung der geschilderten Nachteile, ein einfaches und industriell nutzbares Verfahren zur Isolierung und Reinigung von Oxadimethacrylverbindungen zur Verfügung zu stellen.

Demgemäß wurde ein Verfahren zur Isolierung und Reinigung von Oxadimethacrylverbindungen der allgemeinen Formel I

CH₂=C(A)CH₂-O-CH₂C(B)=CH₂ I

in der A und B ausgewählt sind aus der Gruppe aus -COOR¹, -COR¹, -CONR²R³ und -CN, und R¹, R² und R³ folgende Bedeutung haben:
- R¹: Wasserstoff, Alkyl, unsubstituiertes oder substituiertes Cycloalkyl und Cycloalkyl-alkyl, Hydroxy-alkyl, Amino-alkyl, N-Alkyl-amino-alkyl, N,N-Di-alkyl-amino-alkyl, unsubstituiertes oder substituiertes Aryl und Aryl-alkyl,
- R²,R³: Wasserstoff, Alkyl, unsubstituiertes oder substituiertes Cycloalkyl und Cycloalkyl-alkyl, unsubstituiertes oder substituiertes Aryl und Aryl-alkyl,
durch Kristallisation gefunden, indem man sie aus ihren Lösungen, enthaltend mindestens eine flüssige Kohlenwasserstoffverbindung, ausfällt oder auskristallisiert.

Nach den bisherigen Beobachtungen hat die Art der Reste R¹-, R² und R³ prinzipiell keinen Einfluß auf das erfindungsgemäße Verfahren.

Als Substituenten kommen bevorzugt folgende Reste in Betracht:
- R¹: Wasserstoff;
C₁-C₁₈-Alkyl, darunter vorzugsweise C₁-C₁₂-Alkyl wie Methyl, Ethyl, n-Propyl, i-Propyl, n-Butyl, sek.-Butyl, i-Butyl, tert.-Butyl, n-Pentyl, i-Pentyl, sek.-Pentyl, tert .-Pentyl, neo-Pentyl, n-Hexyl, n-Heptyl, n-Octyl, n-Nonyl, n-Decyl, n-Undecyl, n-Dodecyl und Stearyl, besonders bevorzugt C₁-C₄-Alkyl wie Methyl, Ethyl, n-Propyl, i-Propyl, n-Butyl, i-Butyl, sek.-Butyl, tert.-Butyl;
C₃-C₈-Cycloalkyl wie Cyclopropyl, Cyclopentyl, Cyclohexyl, Cycloheptyl, Cyclooctyl, 4-Methylcyclohexyl, 4-Methoxy-cyclohexyl, 2,4,6-Trimethylcyclohexyl;
C₃-C₈-Cycloalkyl-C₁-C₅-alkyl wie Cyclopropylmethyl, Cyclopentylmethyl, Cyclohexylmethyl, Cyclopropylethyl, Cyclopentylethyl, Cyclohexylethyl, Cyclopropylpropyl, Cyclopentylpropyl, Cyclohexylpropyl, Cyclopentylbutyl, Cyclohexylbutyl, Cyclopentylpentyl, Cyclohexylpentyl, Cyclooctylpentyl;
Hydroxy-C₁-C₅-alkyl wie Hydroxymethyl, 2-Hydroxyethyl, 2-Hydroxypropyl, 3-Hydroxypropyl, 4-Hydroxybutyl, 5-Hydroxypentyl, 2, 2-Dimethyl-3-hydroxypropyl;
Amino-C₁-C₅-alkyl wie Aminomethyl, 2-Aminoethyl, 3-Aminopropyl, 4-Aminobutyl, 5-Aminopentyl;
N-C₁-C₄-Alkyl-amino-C₁-C₅-alkyl wie N-Methylaminomethyl, 2-(N-Methylamino)ethyl, 3-(N-Methylamino)propyl, 4-(N-Methylamino)butyl, 5-(N-Methylamino)pentyl, N-Ethylaminomethyl, N-n-Propylaminomethyl, N-n-Butylaminomethyl;
N,N-Di(C₁-C₄-Alkyl)amino C₁-C₅-alkyl wie N,N-Dimethylaminomethyl, 2-(N,N-Dimethylamino)ethyl, 3-(N,N-Dimethylamino)propyl, 4-(N,N-Dimethylamino)butyl, 5-(N,N-Dimethylamino)pentyl, N,N-Diethylaminomethyl, N,N-Di(n-Propyl)aminomethyl, N,N-Di(i-Propyl)aminomethyl, N,N-Di-(n-Butyl)aminomethyl, N-Ethyl-N-methyl-aminomethyl, N-Methyl-N-propyl-aminomethyl;
C₆-C₁₈-Aryl wie Phenyl, Naphthyl, Anthracenyl, Phenantrenyl, Azulenyl, Biphenylenyl, Triphenylenyl, bevorzugt Phenyl, wobei die Arylreste bis zu drei der unter R⁴ genannten Gruppen tragen können;
C₆-C₁₈-Aryl-C₁-C₄-alkyl, bevorzugt Phenyl-C₁-C₄-alkyl wie Benzyl, 2-Phenylethyl, 3-Phenylpropyl, 4-Phenylbutyl, besonders bevorzugt Benzyl, 2-Phenylethyl, 3-Phenyl_ propyl, wobei die Arylgruppen bis zu drei der unter R⁴ genannten Gruppen tragen können;
- R², R³: C₁-C₁₈-Alkyl wie bei R¹ genannt, darunter besonders bevorzugt C₁-C₄-Alkyl wie Methyl, Ethyl, n-Propyl, i-Propyl, n-Butyl, sek.-Butyl, i-Butyl, tert.-Butyl;
C₃-C₈-Cycloalkyl wie Cyclopropyl, Cyclopentyl, Cyclohexyl, Cycloheptyl, Cyclooctyl, 4-Methylcyclohexyl, 2,4,6-Trimethylcyclohexyl;
C₆-C₁₈-Aryl wie bei R¹ genannt, vorzugsweise Phenyl, welches bis zu drei der unter R⁴ genannten Gruppen tragen kann;
C₆-C₁₈-Aryl-C₁-C₄-alkyl wie bei R¹ genannt, vorzugsweise Phenyl-C₁-C₄-alkyl, besonders bevorzugt Benzyl, 2-Phenylethyl, 3-Phenylpropyl, wobei die Phenylgruppe bis zu drei der unter R⁴ genannten Gruppen tragen kann;
- R⁴: Halogen wie Fluor, Chlor, Brom und Iod, C₁-C₂₂-Alkyl wie Methyl, Ethyl, n-Propyl, i-Propyl, n-Butyl, sek.-Butyl, i-Butyl, tert.-Butyl, n-Pentyl, i-Pentyl, sek.-Pentyl, tert.-Pentyl, neo-Pentyl, n-Hexyl, n-Heptyl, n-Octyl, n-Nonyl, n-Decyl, n-Undecyl, n-Dodecyl, n-Tridecyl, n-Tetradecyl, n-Pentadecyl, n-Hexadecyl, n-Heptadecyl, n-Octadecyl, n-Nonadecyl, n-Eicosyl, n-Heneicosyl und n-Docosyl, vorzugsweise C₁-C₁₂-Alkyl wie Methyl, Ethyl, n-Propyl, i-Propyl, n-Butyl, sek.-Butyl, i-Butyl, tert.-Butyl, n-Pentyl, i-Pentyl, sek.-Pentyl, tert.-Pentyl, neo-Pentyl, n-Hexyl, n-Heptyl, n-Octyl, n-Nonyl, n-Decyl, n-Undecyl, n-Dodecyl und Stearyl, besonders bevorzugt C₁-C₄-Alkyl wie Methyl, Ethyl, n-Propyl, i-Propyl, n-Butyl, i-Butyl, sek.-Butyl, tert.-Butyl;
C₁-C₄-Alkoxy wie Methoxy, Ethoxy, n-Propoxy und n-Butoxy, Carboxy, C₁-C₄-Alkoxy-carbonyl wie Methoxycarbonyl, Ethoxycarbonyl, n-Propoxycarbonyl und n-Butoxycarbonyl, Aminocarbonyl, C₁-C₄-Alkylaminocarbonyl wie Methylaminocarbonyl, Ethylaminocarbonyl, n-Propylaminocarbonyl und n-Butylaminocarbonyl, Di-(C₁-C₄-Alkyl)aminocarbonyl wie Dimethylaminocarbonyl, Diethylaminocarbonyl, Di-(n-Propyl)aminocarbonyl und Di-(n-Butyl)aminocarbonyl, Nitrilo, Nitro, Amino, C₁-C₄-Alkylamino wie Methylamino, Ethylamino, n-Propylamino und n-Butylamino, Di-(C₁-C₄-alkyl)amino wie Dimethylamino, Diethylamino, Di-(n-Propyl)amino und Di-(n-Butyl)amino.

Nach dem erfindungsgemäßen Verfahren fällt oder kristallisiert man Oxadimethacrylverbindungen I aus ihren Lösungen, die mindestens eine (bei Raumtemperatur) flüssige Kohlenwasserstoffverbindung enthalten. Dabei kann diese Kohlenwasserstoffverbindung bereits von Anfang an in diesen Lösungen vorhanden sein oder erst später hinzugefügt werden.

In der Regel verwendet man das erfindungsgemäße Verfahren zur Aufarbeitung von Reaktionsgemischen, die im allgemeinen neben der Oxadimethacrylverbindung weitere Stoffe wie Ausgangsstoffe, Katalysatoren, Stabilisatoren usw. enthalten können. Natürlich kann man das erfindungsgemäße Verfahren auch dazu verwenden, um aus mehr oder weniger reinen Oxadimethacrylverbindungen reinere Oxidimethacrylverbindungen zu erhalten.

Die, die Löslichkeit herabsetzende Kohlenwasserstoffverbindung, kann man schon zu Beginn der Herstellung der Lösung einsetzen. Zweckmäßig verfährt man in der Regel so, daß man sie erst dann der Lösung mit der Oxadimethacrylverbindung hinzufügt, wenn man den Fällungs- oder Kristallisationsvorgang einleiten möchte.

Als Kohlenwasserstoffverbindungen verwendet man in der Regel solche, deren Siedepunkte im Bereich von 20 bis 200°C, bevorzugt von 35 bis 130°C, liegen, wie aliphatische, cycloaliphatische oder aromatische Kohlenwasserstoffe oder deren Mischungen. Beispielhaft seien genannt n-Pentan, n-Hexan, n-Heptan, n-Octan, n-Nonan, n-Decan sowie deren verzweigte Isomere, Cyclopentan, Cyclohexan, Cycloheptan, Cyclooctan sowie mit C₁-C₄-Alkyl-Gruppen substituierte Cycloaliphaten wie Methylcyclopentan und Methylcyclohexan, oder aromatische Kohlenwasserstoffe wie Benzol, Toluol, o-, m- und p-Xylol.

Die Kohlenwasserstoffverbindung setzt man in der Regel im Gewichtsverhältnis Kohlenwasserstoffverbindung zu Oxadimethacrylverbindung I von 1:1 bis 100:1, bevorzugt von 1:1 bis 10:1, besonders bevorzugt von 2:1 bis 4:1 ein.

Vorzugsweise wählt man die Temperatur zu Beginn der Kristallisation so, daß sie zweckmäßig 5 bis 15°C unterhalb des Siedepunkts der Kohlenwasserstoffverbindung liegt, um eine möglichst hohe Konzentration an Oxadimethacrylverbindung I in der Kohlenwasserstoffverbindung zu erhalten. Man kann selbstverständlich auch bei anderen Temperaturen arbeiten, beispielsweise bei Raumtemperatur. Jedoch arbeitet man im allgemeinen innerhalb des Bereiches von 20 bis 200°C, bevorzugt von 40 bis 130°C, wobei es gegebenenfalls erforderlich sein kann, daß Drücke größer als Atmosphärendruck zur Anwendung gelangen. In der Regel wählt man einen Druckbereich von 70 bis 250 kPa, bevorzugt Atmosphärendruck.

Fügt man die Kohlenwasserstoffverbindung erst zur Einleitung der Kristallisation hinzu, oder ist die Kohlenwasserstoffverbindung nicht oder nur teilweise mit dem entsprechenden Lösungsmittel mischbar, so kann es vorteilhaft sein, das Gemisch vor der Kristallisation nach üblichen Methoden wie Schütteln, Rühren oder mittels Flüssig-Flüssig-Extraktion, intensiv zu durchmischen. Diesen Schritt kann man ein- oder mehrstufig, kontinuierlich oder diskontinuierlich durchführen. Die Temperatur bei diesem Schritt wählt man zweckmäßig in dem oben genannten Bereich von 20 bis 200, bevorzugt von 40 bis 130°C.

Zur Umkristallisation verwendet man im allgemeinen Lösungen, die die Oxadimethacrylverbindungen in Mengen im Bereich von 5 bis 50, bevorzugt von 10 bis 30 Gew.-% enthalten. Als Lösungsmittel verwendet man dabei in der Regel die weiter oben genannten Kohlenwasserstoffverbindungen.

Das anschließende Auskristallisieren der Oxadimethacrylverbindung I nimmt man in der Regel bei Temperaturen von (-80) bis 30°C, bevorzugt von (-30) bis 20°C vor. Das auskristallisierte Produkt trennt man dann wie üblich, beispielsweise durch Filtrieren oder Zentrifugieren, ab und trocknet es in an sich bekannter Weise.

Nach dem Kristallisationsvorgang kann man, bei Vorliegen von zwei oder mehreren flüssigen Phasen, die nicht mit der Kohlenwasserstoffverbindung angereicherte Phase abtrennen und sie einem erneuten Aufarbeitungszyklus zuführen, um auch die restlichen Mengen der Oxadimethacrylverbindung I zu erfassen. Diesen Vorgang kann man beliebig oft wiederholen und kontinuierlich oder diskontinuierlich durchführen.

Die Vorgehensweise bei anderen Lösungen oder Gemischen, die Oxadimethacrylverbindungen enthalten, beispielsweise bei Reaktionsgemischen, die bei der Herstellung von Oxadimethacrylverbindungen anfallen, ist im Prinzip dieselbe wie oben beschrieben. In der Regel entfallen aufwendige Vorreinigungsschritte wie Destillation, Extraktion oder Chromatographie. Dabei hängt der Einsatz solcher Schritte von der vorliegenden Lösung, Mischung oder dem Gemisch ab. Dies trifft in der Regel bei Reaktionsgemischen zu, in denen die Oxadimethacrylverbindung I nicht als Hauptprodukt gebildet wurde. Desweiteren kann es beispielsweise von Vorteil sein, in Reaktionsgemischen die überschüssigen Ausgangsstoffe wie Acrylverbindungen vor der Kristallisation abzutrennen.

Eine besondere Ausführungsform betrifft die Isolierung und Reinigung von Reaktionsgemischen, die bei der Herstellung von Oxadimethacrylverbindungen nach einem der folgenden Verfahren anfallen:
A) Umsetzung einer Acrylverbindung der allgemeinen Formel II

   H₂C=C(A)H II

   in Gegenwart von Sauerstoff, mindestens einem tertiären Amin und vorzugsweise mindestens einem Polymerisationsinhibitor mit Formaldehyd oder einer Formaldehyd-liefernden Verbindung zum Alkohol der allgemeinen Formel III

   H₂C=C(A)CH₂OH III

   und anschließende weitere Umsetzung des Alkohols III,
   b₁) unter Isolierung desselben oder
   b₂) ohne Isolierung desselben
   unter Erhitzen in Gegenwart von Sauerstoff, mindestens einem tertiären Amin und vorzugsweise mindestens einem Polymerisationsinhibitor zur Oxadimethacrylverbindung I, CH₂=C(A)CH₂-O-CH₂C(A)=CH₂, oder
B) Umsetzung des Alkohols III in Gegenwart von Sauerstoff, mindestens einem tertiären Amin und vorzugsweise mindestens einem Polymerisationsinhibitor unter Erhitzen zur Oxadimethacrylverbindung I, CH₂=C(A)CH₂-O-CH₂C(A)=CH₂, oder
C) Umsetzung einer Mischung aus zwei verschiedenen Acrylverbindungen der allgemeinen Formeln II und IIa 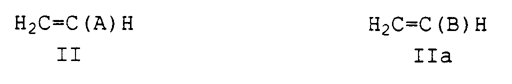 in Gegenwart von Sauerstoff, mindestens einem tertiären Amin und vorzugsweise mindestens einem Polymerisationsinhibitor mit Formaldehyd oder einer Formaldehyd-liefernden Verbindung zu den Alkoholen der allgemeinen Formeln III und IIIa 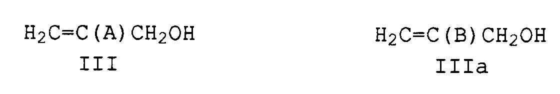 und anschließende weitere Umsetzung mit oder ohne weitere Isolierung der Alkohole III und IIIa mit entweder
   a) dem diese Alkohole enthaltenden Reaktionsgemisch, oder
   b) den isolierten Alkoholen
   unter Erhitzen in Gegenwart von Sauerstoff, mindestens einem tertiären Amin und vorzugsweise mindestens einem Polymerisationsinhibitor zur Oxadimethacrylverbindung I, CH₂=C(A)CH₂-O-CH₂C(B)=CH₂, oder
D) Umsetzung einer Acrylverbindung II in Gegenwart von Sauerstoff, mindestens einem tertiären Amin und mindestens einem Polymerisationsinhibitor mit Formaldehyd oder einer Formaldehyd-liefernden Verbindung zum Alkohol III, und anschließende weitere Umsetzung des isolierten Alkohols III, oder des den nicht isolierten Alkohol III enthaltenden Reaktionsgemisches, mit einem weiteren, davon verschiedenen Alkohol IIIa unter Erhitzen in Gegenwart von Sauerstoff, mindestens einem tertiären Amin und vorzugsweise mindestens einem Polymerisationsinhibitor zur Oxadimethacrylverbindung I, CH₂=C(A)CH₂-O-CH₂C(B)=CH₂, oder
E) Umsetzung einer Mischung aus zwei verschiedenen Alkoholen III und IIIa in Gegenwart von Sauerstoff, mindestens einem tertiären Amin und mindestens einem Polymerisationsinhibitor unter Erhitzen zur Oxadimethacrylverbindung I CH₂=C(A)CH₂-O-CH₂C(B)=CH₂.

Die für diese Verfahren benötigten Acrylverbindungen II sind entweder käuflich oder man erhält sie beispielsweise durch Veresterung, Umesterung, Amidierung oder Aminolyse nach an sich bekannten Methoden (s. H.Rauch-Puntigam et al., Chemie, Physik und Technologie der Kunststoffe, Bd.9, Springer Verlag, Berlin, 1967) aus den entsprechenden leicht zugänglichen Acrylvorstufen wie Acrylsäure und deren bekannte Derivate.

Die entsprechenden Alkohole III sind entweder bekannt (s. EP-B 184 731) oder nach einem der oben angegebenen Verfahren aus den Acrylverbindungen II zugänglich.

Den Formaldehyd kann man gasförmig, flüssig, beispielsweise als wäßrige Lösung wie Formalin oder in Form einer alkoholischen Lösung, oder in fester Form, zum Beispiel als Paraformaldehyd, Trioxan, Tetroxocan oder als Halbacetal einsetzen.

Als tertiäre Amine kommen offenkettige aliphatische oder cyclische tertiäre Amine in Betracht wie Trimethylamin, Triethylamin, Tri-n-propylamin, Triisopropylamin, Tri-n-butylamin, Triisobutylamin, Tri-n-pentylamin, Methyldiisopropylamin, N,N-Diethylisopropylamin, N,N-Dimethylethylamin, N,N-Dimethylisopropylamin, Tri-2-ethylhexylamin, N-Methyldiethylamin, N,N-Dimethyl-n-propylamin, N,N-Dimethyl-n-butylamin, N,N-Dimethyl-isobutylamin, N,N-Dimethyl-(2-ethylhexyl)amin, N,N-Diisopropyl-(2-ethylhexyl)amin, N,N-Di-nbutyl-(2-ethylhexyl)amin, N-Methyl-di-(2-ethylhexyl)amin, N-n-butyl-(2-ethylhexyl)amin, N-Isobutyl- di-(2-ethylhexyl)amin, Chinuclidin und 1,4-Diazabicyclo[2.2.2]octan (DABCO®), bevorzugt Chinuclidin und DABCO®, besonders bevorzugt DABCO®.

Als Polymerisationsinhibitoren verwendet man in der Regel die üblichen wie Hydrochinon, Hydrochinonmonomethylether, p-Benzochinon, Phenol, 2,6-Dimethylphenol, 2,6-Di-tert.-Butylphenol, Methylenblau, Diphenylamin, Cu-(II)-oleat, Fe-(III)-acetylacetonat, Brenzcatechin, bevorzugt Hydrochinonmonomethylether und Hydrochinonmonoethylether.

Den Sauerstoff kann man in reiner Form oder in Form eines Gemisches mit nicht reaktiven Gasen, bevorzugt Luft, über oder durch das Reaktionsgemisch leiten.

Bei der Umsetzung der Acrylverbindung II, bzw. der Mischung II und IIa, zur Oxadimethacrylverbindung I über die Alkoholverbindung III, setzt man in der ersten Stufe die Acrylverbindung II bzw. die Mischung II und IIa und den Formaldehyd im allgemeinen im Molverhältnis Acrylverbindung II bzw. Mischung II und IIa zu Formaldehyd von 1:1 bis 8:1, vorzugsweise von 1,0:1 bis 2,5:1, ein.

Das tertiäre Amin verwendet man hierbei vorzugsweise im Molverhältnis Formaldehyd zu Amin von 1:1 bis 200:1, bevorzugt 2:1 bis 100:1, besonders bevorzugt 4:1 bis 50:1.

Den Polymerisationsinhibitor setzt man in der Regel in Mengen von 10 bis 1000 mg pro kg Acrylverbindung II bzw. Mischung II und IIa ein.

Die Menge des Sauerstoffs liegt in der Regel im Bereich von 0,01 bis 100, bevorzugt von 0,1 bis 20 l/h pro kg Acrylverbindung II bzw. Mischung II und IIa. Verwendet man Luft als Sauerstofflieferanten, so wählt man die Gasmenge im allgemeinen im Bereich von 0,01 bis 1000, bevorzugt von 1 bis 250 l/h pro kg Acrylverbindung II bzw. Mischung II und IIa.

Im allgemeinen arbeitet man bei Temperaturen von 10 bis 100°C, bevorzugt von 40 bis 80°C, besonders bevorzugt von 60 bis 75°C. Desweiteren führt man die Reaktion in der Regel unter Atmosphärendruck durch. Sie kann jedoch auch bei vermindertem oder erhöhtem Druck vorgenommen werden. Ein Arbeiten unter Druck ist vor allem dann angezeigt, wenn man die Umsetzung bei Temperaturen oberhalb von 80°C vornimmt.

Des weiteren führt man die Reaktion in der Regel ohne Lösungsmittel durch. Jedoch kann man die Umsetzung auch in Gegenwart eines geeigneten Lösungsmittels wie ein C₅-C₈-Al-kan, bevorzugt n-Pentan, n-Hexan, n-Heptan, n-Octan, i-Octan, ein Carbonsäureester wie Ethylacetat, sowie ein aromatisches Lösungsmittel wie Benzol, Toluol und Xylole, besonders bevorzugt n-Hexan, i-Octan und Toluol, oder deren Mischungen, durchführen.

Die Reaktionszeit hängt hauptsächlich von der Reaktionstemperatur ab. Sie liegt im allgemeinen im Bereich von 1 bis 6 h.

Den bei dieser Reaktion gebildeten Alkohol III, bzw. die Mischung III und IIIa kann man nach den üblichen Aufarbeitungsmethoden wie Destillation oder Chromatographie isolieren.

In der zweiten Stufe, ausgehend vom Alkohol III, bzw. von der Mischung III und IIIa, wählt man in der Regel Art und Menge des Amins, des Polymerisationsinhibitors sowie des Lösungsmittels wie in der ersten Stufe. Die Menge des Sauerstoffs liegt in der Regel im Bereich von 0,01 bis 1000, bevorzugt von 0,1 bis 50 l/h pro kg Alkoholverbindung III bzw. Mischung III und IIIa. Verwendet man Luft als Sauerstofflieferanten, so wählt man die Gasmenge im allgemeinen im Bereich von 0,1 bis 1000, bevorzugt von 1 bis 500 l/h pro kg Alkoholverbindung III bzw. Mischung III und IIIa.

Die Umsetzung der zweiten Stufe (Alkohol III zu Oxadimethacrylverbindung I) nimmt man im allgemeinen bei einer Temperatur im Bereich von 100 bis 200°C, bevorzugt von 100 bis 150°C, und bei einem Druck, der in der Regel im Bereich von 70 bis 300 kPa liegt, vor, bevorzugt arbeitet man jedoch unter Atmosphärendruck.

Das während der Reaktion anfallende Reaktionswasser kann man in der Regel durch Destillation, bevorzugt durch Rektifikation, aus dem Reaktionsgemisch entfernen.

Zweckmäßig kann man dabei dem Reaktionsgemisch ein Schleppmittel zusetzen. Hierfür eignen sich beispielsweise aliphatische, cycloaliphatische und aromatische Kohlenwasserstoffe wie n-Hexan, n-Heptan, i-Octan, Benzol, Toluol, Xylol, Cyclohexan, sowie Carbonsäureester wie Essigsäureethylester, oder die vor der Reaktion nicht abgetrennte Acrylverbindung II. Den Siedepunkt der Schleppmittel wählt man im allgemeinen im Bereich zwischen 80 und 200°C.

Die Reaktionszeit ist von den üblichen Parametern wie Temperatur, Druck und den Mengen der Ausgangsstoffe abhängig und liegt in der Regel im Bereich von 4 bis 12 h.

Führt man die Reaktionen ausgehend von der Acrylverbindung II, bzw. der Mischung II und IIa, einstufig aus, d.h. ohne Isolierung des Alkohols III bzw. der Mischung III und IIIa, so trennt man zweckmäßigerweise die im überschuß noch vorhandene Acrylverbindung II, bzw. die Mischung II und IIa, vor der Weiterreaktion zur Oxadimethacrylverbindung I ab, beispielsweise durch Destillation. Dies kann jedoch auch nach der Reaktion zur Oxadimethacrylverbindung I erfolgen.

Besonders bevorzugt wird bei Verwendung eines der Verfahren A), C) und D) die in der Regel im Überschuß vorliegende Acrylverbindung II bzw. IIa abdestilliert, bevor mit der Isolierung und Reinigung durch Kristallisation begonnen wird. Ebenso ist es zweckmäßig, das sich bei den Verfahren A) bis E) gebildete Reaktionswasser vor der Kristallisation beispielsweise durch Destillation abzutrennen.

Nach den bisherigen Beobachtungen erhält man nach der Aufarbeitung durch Kristallisation die Oxadimethacrylverbindungen I in einer Reinheit ≧ 90 %.

Die nach dem erfindungsgemäßen Verfahren erhaltenen Oxadimethacrylverbindungen kann man nach an sich bekannten Methoden (s. US-A 4,889,948) polymerisieren. Desweiteren kann man sie beispielsweise auch durch 1,6-intra-intermolekulare Cyclopolymerisation zu cyclischen Ethern, die z.B. in der Zahnmedizin Verwendung finden, umsetzen (s. Polymer Preprints, 31(1), 1990, 503) .

### Beispiele

### Beispiel 1

### Darstellung von 2,2' [Oxybis(methylen)]bis-2-propensäure-di-methylester ausgehend von Methylacrylat und Formaldehyd

### a) mit chromatographischer Aufarbeitung

Eine Mischung aus 860 g (10 mol) Methylacrylat, 150 g (5 mol) Paraformaldehyd, 56 g (0,5 mol) DABCO® (1,4-Diazabicyclo[2.2.2]octan) und 172 mg Hydrochinonmonomethylether wurde während 1,75 h auf 75°C erhitzt, wobei gleichzeitig 10 l/h Luft durch die Mischung geleitet wurden. Dann wurde unter weiterem Erhitzen überschüssiges Methylacrylat soweit abdestilliert, bis die Temperatur des Reaktionsgemisches im Sumpf 135°C betrug. Anschließend wurde das bei der Reaktion entstandene Reaktionswasser während 4h bei 135°C abdestilliert, wobei als azeotropes Schleppmittel noch vorhandenes Methylacrylat diente. Danach erhielt man durch präparative Säulenchromatographie des Destillationsrückstandes an Silikagel mit Ethylacetat/Hexan (20/80) als Elutionsmittel
64 g (12 %) 2-Hydroxymethylacrylsäuremethylester,
460 g (86 %) 2,2' [Oxybis (methylen) ]bis-2-propensäure-dimethylester und
2,5 g (2 %) höhere Homologe der Oxadimethacrylverbindung.

### b) Aufarbeitung durch Kristallisation

Der Versuch aus 1a) wurde mit den gleichen Mengen unter gleichen Bedingungen wiederholt. Jedoch wurde nach der Abdestillation des Reaktionswassers auf 60°C abgekühlt, mit 1400 g n-Hexan versetzt und eine Stunde bei 60°C gerührt. Danach wurde die untere Phase abgetrennt und die obere Phase unter Rühren auf 0°C abgekühlt. Hierbei fiel ein Teil des Dimethylesters in Form von feinen, weißen Kristallen aus. Nach dem Abfiltrieren, Waschen mit 300 ml 0°C kaltem n-Hexan und Trocknen bei 25°C/100 mbar, wurden 482 g (90 %) des Dimethylesters in einer Reinheit von 95 % erhalten. Konzentrieren der Mutterlauge im Vakuum, erneutes Versetzen mit n-Hexan bei 60°C und wiederholte Kristallisation lieferten weitere 21 g des Dimethylesters in einer Reinheit von 92 %.

### Beispiel 2

### Darstellung von 2,2' [Oxybis (methylen) ]bis-2-propensäure-dicyclohexylester

### a) chromatographische Aufarbeitung

Eine Mischung aus 308 g (2 mol) Cyclohexylacrylat, 30 g (1 mol) Paraformaldehyd, 11,2 g (0,1 mol) DABCO® und 172 mg Hydrochinonmonomethylether wurde während 6 h auf 75°C erhitzt, wobei gleichzeitig 10 l/h Luft durch die Mischung geleitet wurden. Dann wurde unter weiterem Erhitzen überschüssiges Cyclohexylacrylat soweit abdestilliert, bis die Temperatur des Reaktionsgemisches im Sumpf 135°C betrug. Anschließend wurde das bei der Reaktion entstandene Reaktionswasser während 18 h bei 135°C abdestilliert, wobei als azeotropes Schleppmittel Isooctan diente. Danach erhielt man durch präparative Säulenchromatographie des Destillationsrückstandes an Silikagel mit Ethylacetat/Hexan (20/80) als Elutionsmittel
46 g (26 %) 2-Hydroxymethylacrylsäurecyclohexylester,
127 g (73 %) 2,2' [Oxybis (methylen) ]bis-2-propensäure-dicyclohexylester und
2 g (1 %) höhere Homologe der Oxadimethacrylverbindung.

### b) Aufarbeitung durch Kristallisation

Eine Mischung aus 308 g (2 mol) Cyclohexylacrylat, 30 g (1 mol) Paraformaldehyd, 11,2 g (0,1 mol) DABCO® und 20 mg Hydrochinonmonomethylether wurde während 6 h auf 75°C erhitzt, wobei gleichzeitig 10 l/h Luft durch die Mischung geleitet wurden. Dann wurde im Vakuum überschüssiges Cyclohexylacrylat abdestilliert. Anschließend wurde das bei der Reaktion entstandene Reaktionswasser unter langsamem Erhitzen bis 135°C während 12 h abdestilliert, wobei als azeotropes Schleppmittel Isooctan diente. Nach dem Abdestillieren des Reaktionswassers wurde auf 60°C abgekühlt, mit 340 g n-Hexan versetzt und eine Stunde bei 60°C weitergerührt. Danach wurde die untere Phase abgetrennt und die obere Phase unter Rühren auf 0°C abgekühlt. Hierbei fiel ein Teil des Dicyclohexylesters in Form von feinen, weißen Kristallen aus. Nach dem Abfiltrieren, Waschen mit 100 ml 0°C kaltem n-Hexan und Trocknen bei 25°C/100 mbar, wurden 142 g (81 %) des Dicyclohexylesters in einer Reinheit von 96 % erhalten. Konzentrieren der Mutterlauge im Vakuum, erneutes Versetzen mit n-Hexan bei 60°C und wiederholte Kristallisation lieferten weitere 9 g des Dicyclohexylesters in einer Reinheit von 92 %.

## Patentansprüche

1. Verfahren zur Isolierung und Reinigung von Oxadimethacrylverbindungen der allgemeinen Formel I
CH₂=C(A)CH₂-O-CH₂C(B)=CH₂ I
in der A und B ausgewählt sind aus der Gruppe aus -COOR¹, -COR¹, -CONR²R³ und -CN, und R¹, R² und R³ folgende Bedeutung haben:
R¹ Wasserstoff, Alkyl, unsubstituiertes oder substituiertes Cycloalkyl und Cycloalkyl-alkyl, Hydroxyalkyl, Amino-alkyl, N-Alkyl-amino-alkyl, N, N-Dialkyl-amino-alkyl, unsubstituiertes oder substituiertes Aryl und Aryl-alkyl,
R²,R³ Wasserstoff, Alkyl, unsubstituiertes oder substituiertes Cycloalkyl und Cycloalkyl-alkyl, unsubstituiertes oder substituiertes Aryl und Aryl-alkyl,
dadurch gekennzeichnet, daß man sie aus ihren Lösungen, enthaltend mindestens eine flüssige Kohlenwasserstoffverbindung, ausfällt oder auskristallisiert.
